# EUROPEAN PATENT SPECIFICATION

(11) **EP 3 875 033 B1**
(45) Date of publication and mention of the grant of the patent: **28.09.2022**
(21) Application number: 20160324.8
(22) Date of filing: 02.03.2020
(51) Int. Cl.: A61B 6/10, A61B 6/00, A61B 8/00, G06T 7/70, G01R 33/54

(54) **METHOD FOR SUPERVISING AUTOMATED IMAGING MODALITY MOVEMENT IN A MEDICAL SCENE**
VERFAHREN ZUR ÜBERWACHUNG DER AUTOMATISIERTEN BEWEGUNG DER BILDGEBUNGSMODALITÄT IN EINER MEDIZINISCHEN SZENE
PROCÉDÉ POUR SUPERVISER UN MOUVEMENT DE MODALITÉ D'IMAGERIE AUTOMATISÉ DANS UNE SCÈNE MÉDICALE

(43) Date of publication of application: 08.09.2021
(73) Proprietor: AGFA NV, 2640 Mortsel (BE)
(72) Inventor: NEBOSIS, Rainer, 81539 München (DE)
(74) Representative: Verbrugghe, Anne Marie L.

(56) References cited:
- WO-A1-2010/150148
- DE-A1-102018 209 691
- US-A1- 2003 230 724
- US-A1- 2019 311 490

## Description

### Technical Field

The present invention relates to a method to supervise and control the safety aspects of automated movement of a medical imaging modality that is controlled by means of an automated movement program, and that is not supervised by a human user.

### Background of the invention

In preparation of any imaging examination of a patient, a number of labour intensive steps have to be performed in order to eventually obtain the intended configuration and arrangement of the combination of the device and the patient. The overhead associated with the manual interventions that need to be performed by an operator may impair the effectiveness of the utilisation of the medical imaging modality equipment. The medical industry has always been searching for improvements to the techniques and procedures which are involved in these preparative steps, and this in order to relieve the operator (which is in most cases a radiographer) as much as possible from the repetitive and burdensome tasks.

As a first step, the patient will need to be positioned in or on the system. This positioning step is carried out with a prime focus on an optimal positioning of the region of interest (that is located somewhere in the patient) with respect to the position of the imaging detector(s) and the imaging source. But at the same time, attention is given to the comfort of the posture that the patient has to assume for the duration of the medical image acquisition itself, making sure that the patient can sustain his position sufficiently long in comfort.

Bringing the patient into the desired position may involve a number of preparative steps such as for instance the lowering of the surface of a positioning table such that the patient can initially mount the table top in a sitting position and then move into a horizontal lying position. In another example, the operator will assist the patient to assume a standing position at the correct distance from the surface of a wall-stand detector, thereby ensuring that his arms are not obstructing the imaging detector.

As a second step, the active components (the radiation source and imaging detector) of the imaging modality are positioned close to or around the region of interest within the patients' body. This alignment step serves to fine-tune the positioning of the active components with respect to the patient, and will ensure that certain distances between the patient and the components are optimized or at least respected in order to ensure an acquisition that meets the quality criteria. This step will in most of the cases involve the manipulation by the operator of either the detector and the imaging source, or both.

While certain modalities are handheld (such as for instance ultrasound devices, where the transducer is handheld) others comprise components which have bulky dimensions and may be very heavy. These larger imaging components may be suspended in a fixed gantry (such as for instance is the case with a CT or MRI-system), or may be supported by fixed mounts, floor stands, mobile stands and carts, or ceiling suspensions that may be equipped with segmented arms supporting these devices. The segmented arms offer the required flexibility in positioning the heavy components while being suspended in the room, they essentially allow repositioning of such a component with limited force.

In general, the positioning of movable components may thus require some force from the operator. The burden of requiring usage of force to move some of the heavier imaging components may be relieved by foreseeing power assistance for certain movements. It is for instance very common for mobile X-ray carts to be motorized, because the weight of such systems would not allow positioning and operation of such a device by a single person. To be practical, it is therefore a requirement that the positioning of the components would require the least of efforts possible for the operator.

A further improvement for the advantage of the operator that can be implemented on imaging modalities with moving components is the automation of such moving components. A prerequisite for this is obviously that the components are at least partially motor driven. In a typical X-ray modality, it could be envisaged that the movement of the table, overhead ceiling suspension of the X-ray tube (or tube crane), the floor-mounted wall stand could be controlled remotely or automatically. This functionality would open the door to appealing and time-saving positioning solutions that would relief the operator from certain repetitive modality component positioning tasks. This could be achieved by programming the motorized movement of the imaging components, allowing complex computer steered modality component movement patterns.

A practical example of the application of such an automated movement could be the automatic return of the imaging modality to an initial (default) position after the completion of a last imaging exam, whereby the patient and operator would have left the room. In such a scenario, the imaging modality would automatically reposition all its components to their default starting position. Another example could be the automatic preparation of the X-ray modality for a wall-stand exposure, whereby the active imaging detector would be located in the wall-stand, and whereby the X-ray tube would be oriented towards the wall-stand detector and this at a predefined distance of for instance 150cm.

Although that the concept of automated movement may be very appealing, stringent safety considerations are to be taken into account. In practice, the automated movement would only be allowable if it can be guaranteed that no person could be injured by such movement, or that the automated movement would not inflict damage to the system caused by collision with foreign objects or by self-collision. In order to prevent collision with any person in the room, it is for instance required to ascertain that no-one is present in the X-ray room before any automated movement would be initialised. The validation of this requirement could be done by a person (who then would have to give the start signal and supervise during the movement) or by technological means (such as automated analysis of camera images of the X-ray room).

In the art, different approaches to resolve these safety considerations with technological means are described, but none of them appear to be fail-proof.

A collision detection system for a medical imaging modality that would be aware of its surroundings, and that could monitor its own proper functioning, would therefore be a much wanted feature, as it would offer potential to reduce the workload for the operator in practice. Such a system should then be aware of the presence of any persons or foreign objects in the room.

### Summary of invention

The present invention provides a system to supervise an automated movement of at least one movable component of an imaging modality within a medical scene to prevent collision between said movable component and other objects within said medical scene, said movable component providing a data stream comprising at least one positional parameter determining said movable components' geometric state in the medical scene, the system comprising, an interface adapted to read out said positional parameter from said data stream from said movable component, a memory storing: dynamic model data representing geometric knowledge of said at least one movable component in the medical scene as a function of said positional parameter of said movable component in said data stream, stationary model data representing geometric knowledge on all non-movable objects in the medical scene, a medical scene map, at least one distance detector fixed at a known position in said medical scene, said known position is stored in said medical scene map, said distance detector providing at least one measured distance between said distance detector and an observed object along a detection axis of said distance detector, a processor configured to: calculate said medical scene map from said dynamic model data in combination with said at least one positional parameter, and from said stationary model data, calculate a calculated distance between said stored position of said distance detector in said medical scene map and a first object along said detection axis of said distance detector in said medical scene map, compare said at least one measured distance with said calculated distance, such that when the difference between said at least one measured distance and said calculated distance exceeds a threshold value, a trigger signal is sent to said imaging modality.

The system of the invention is conceived to supervise automated movement of movable image modality components in a medical scene.

In the context of this invention, a geometric state of a movable component within the medical scene is fully characterised by its position, orientation and its own dimensions within said medical scene. The geometric state of the movable component is thus a particular state that the component can assume in reality.

In the context of this invention, geometric knowledge has to be understood as the knowledge that describes all different geometric states that the movable component can assume taking into account the limitations of its design, and for instance also the limitations to its movements by neighbouring or even connected components. The geometric knowledge is preferably expressed as a model that describes the different positions, orientations and shapes (or dimensions) that the component can assume taking into account its design characteristics and limitations.

In the context of this invention, a medical scene has to be understood as the modality room wherein a limited number of mostly known objects are present, of which most of them are the different parts of the imaging modality system itself. These different imaging modality parts consist of either non-movable components and movable components. The non-movable components are imaging modality parts that do not move during the operation of the modality, such as for instance is the case for the X-ray generator cabinet, or a fixed wall-stand base. The movable components are the individual imaging modality parts that can assume different positions, orientations and/or shapes. The same medical scene may comprise exceptionally a number of unknown objects that do not necessarily contribute to the functioning of the imaging modality, or even persons. Since the presence of the latter is rather an exception than a rule, it will be possible for the supervising system to make certain estimations about the probability to encounter certain of these unknown objects or persons, and to filter out these "alien" object detections when desired by the application.

In the context of this invention, the automated movement of at least one modality component concerns a motor driven or powered movement (e.g. hydraulically powered) of an element of, or of the whole component that supports an operator with the moving and positioning of an imaging modality component before or during the image acquisition. The automated movement can thus be considered as any movement functionality (linear, rotation, lifting, ...) or partial movement functionality that an imaging modality component can support. The movement functionality may thus for instance be limited to a rotation around a joint of a motorized arm, a linear movement along a track (such as for instance an extension of a robotic arm), or alike.

A requirement for the system of the invention is however that the movement of the modality component in question can be followed, measured or monitored through a measurable positional parameter that is provided to the system of the invention by means of a data stream. In other words, the movement functionality and more particularly the movement state should be fully described by one or more positional parameter values for each state or position the component can assume. The parameter values determine the different degrees of freedom that may be associated with the modality component.

In practice, the positional parameter is a parameter that characterises a certain movement of a component. The following examples may be considered: the table height is a positional parameter for the up-down movement of an X-ray table, the gantry angle is a positional parameter for the isocentric rotation of a C-arm based mobile X-ray system, the x-axis position (in cm or mm) is a positional parameter for the longitudinal movement in X-direction of a ceiling suspension for an X-ray tube, ...

In any case, there should be an unambiguous relationship between said positional parameters and the geometric state (characterised by its position, orientation and its own dimensions) of the modality component. The positional parameter value may represent a displacement (in which case the value represents a distance), or another geometric parameter such as a rotation angle.

One modality component may be attributed more than one positional parameter. This is the case when the component can move according to more than one degree of freedom when performing its movement. A modality component may for instance extend a shaft in a linear direction, and at the same time perform a rotation around its own axis (this is the case for a ceiling suspended X-ray tube mount). In this case, both movements are interlinked because of the physical connection between the moving components: the joint. The position of an attached object (that is for instance attached to one end of such a combination of components) will then depend on the combination of the multiple positional parameters of the individual modality components involved. The different possible movements in combination with their respective positional parameters are described as a model (and stored as model data) that represent the geometric knowledge about the movable component within the medical scene.

The read-out of the positional parameter values should preferably be presented by the component to the system of this invention as a digital value that is accessible via a digital data stream. The digital data stream of positional parameter values should be understood as a continuously accessible set of digital data representing the actual position or geometric state of the modality component in question. This digital data stream is thus a continuously updated value that is accessible to the collision detection system of the invention through a digital interface, such that the system can poll the interface for the positional parameter value on demand.

It is clear that all considered component movements have their proper intrinsic limitations or constraints regarding their movement potential; rotation angles may be limited by the way certain joints are constructed, and certain telescopic components may only slide or extend up to limited lengths. This information is captured as geometric knowledge in the model data for each movable component. Certain movements of modality components may however also interfere with neighbouring components due to their physical dimensions causing physical interference with parts of other moving components or fixed objects in the room. It is thus clear that the geometric knowledge of the entire imaging modality is much more complex in comparison with the geometric knowledge of a single component.

Therefore, in order to capture all the limitations and potential physical interactions for the movements of all modality components in the medical scene, a virtual medical scene map can be created which is a virtual representation of the actual state of the modality and its surroundings. The medial scene map puts the different components in their respective geometric states in a virtual representation (in the computers' memory) such that it simulates the positions of these components in real space. By doing so, potential interactions between components (collisions) can be detected or simulated in advance. The actual state of the modality can thus be virtually simulated in a medical scene map by superposition of all geometric states of the constituent components. This medical scene map, virtual medical scene map or virtual map should represent both the movable modality components as well as the positions of the immobile or fixed modality components in the medical scene.

In order to be able to calculate this medical scene map, the model or so-called modality model has to be available comprising the knowledge of all the physical characteristics of the different modality components. These characteristics comprise the physical dimensions of the components, their default positions, their intrinsic limitations or constraints of their movements, the relations between the positional parameter values and their position or orientation in the medical scene or in relation to another modality component (for instance when one component is attached to another component), and for instance the movement speeds that may be achieved by the different modality components when their movement mechanism is activated. Any mechanical CAD software or modelling software, or by extension basically any programming environment are known methods to implement and describe such models.

The modality model or model will be expressed as a combination of computer code and/or computer data. This code and data can be stored as such in digital format in a computer memory, for it to be accessed by a processor that can execute the code and process the data. In the context of our invention, this computer code and data expressing the modality model is called "model data". It consists of two distinct parts; the stationary model data and the dynamic model data. The stationary model data thus represents the geometric knowledge from the non-moving components in the medical scene, whereas the dynamic model data represents the geometric knowledge from the movable components in the medical scene.

As mentioned above, a distinction is made between a stationary model and a dynamic model. The stationary model comprises the model data for all immobile or fixed components and only describes the position and location of these immobile or fixed components in the medical scene. No variable parameters will have an impact on the position and location of these immobile components in the medical scene. The dynamic model will however describe the movable components, wherein their orientation and positions are described as a function of their respective attributed positional parameters.

The virtual medical scene map is calculated based on the knowledge stored in these two models (stationary and dynamic), taking into account the various positional parameters that are read out from the imaging modality. The result is that a virtual medical scene map can be calculated for each set of positional parameters, providing a simulation or a virtual representation of the modality in a certain configuration state. The configuration state of an imaging modality is thus the sum (or superposition) of the geometric states of all components, given their respective positional parameters that are applied to the modality model.

The modality model may thus be applied for different purposes. It may be used to make simulations of different modality states of the system that are calculated as a medical scene map, based on the input of any set of positional parameters that are fed into the modality model. By doing so, these simulations allow for instance to verify whether a certain combination of positional parameters would lead to a (self-)collision between different modality components, or with other known objects in the room. A collision can be identified by detecting an overlap of two or more modality components in the virtual medical scene map.

Furthermore, the calculation of this virtual representation of the modality allows to verify whether or not certain components in the model risk to overlap in case of -for instance- a continued movement in a certain direction of a component. These calculations allow thus to predict or anticipate collisions or self-collisions for movements of modality components on planned or programmed trajectories. An overlap of two components in the virtual map namely represents a (virtual) collision between said two modality components for the configuration state of the modality. In other words, simulations may be performed by adapting positional parameters and feeding them into the medical scene model in order to verify whether a collision would occur between certain modality components or not. This way, the system can for instance anticipate collisions by extending positional parameter values along their expected course, and calculating at which point the positional parameter in question will cause a collision, self-collision or a halt due to reaching its design limits.

Another application for calculations of a virtual medical scene map is for instance the calculation (or the prediction) of a distance between two points on a known surface of the modality system, and this for a particular configuration state. The calculation of a virtual medical scene map for a particular configuration state will thus allow the prediction of the distances from a particular point on the surface of a modality component to any other known surface in the model. When calculating distances from one point towards multiple directions, it is possible to reconstruct or simulate a depth image for a certain field of view from the perspective of the point. This simulated depth image could then be compared with an actual depth image recorded by for example a depth camera that would be physically mounted on said particular point on the surface of the modality, in order to validate that the measured result (measured depth image) from the depth camera falls within the tolerances of the simulated depth image, in order to conclude that the supervision system operates appropriately. The latter application would thus allow the supervision system to perform a closed-loop self-checking function that omits the need for a secondary validation circuit to check the proper operation of the system.

As a conclusion, based on this virtual representation of the system in the memory of the computer, it will be possible to assess whether there is a risk for collisions of a considered modality component with another object. And at the same time, it will be possible to pre-calculate an expected depth image from a chosen point for a particular configuration state of an imaging modality. The latter technique allows the supervision system to perform a fundamental self-checking routine ensuring proper validation of the system performance.

Based on the above concepts, an anticipative collision detection system is presented here, that is capable of predicting a foreseeable collision in the assumption that the movement course of the modality components would not be adjusted. It also can detect the presence of foreign objects (or persons) to the medical scene, and take the appropriate action in case of risk for a collision.

In addition to this, a self-checking circuit for this system is presented that at chosen intervals validates the proper operation of the collision detection system itself. This solution is very desirable as it removes the need for a secondary circuit or system to oversee the proper operation of the detection system itself (which is an industry requirement for automatically moving systems). Moreover, the described method is the most straight forward and therefore the safest way to check the system.

One of the main advantages of the collision sensor system is that it can supervise proper functioning of the imaging modality component movements, but also itself; by confirming the locations of certain fixed objects in the field of view of the scanning detector, the supervising system can affirm itself to be functioning correctly.

The embodiments of the systems and methods described herein may be implemented in hardware or software, or a combination of both. However, preferably, these embodiments are implemented in computer programs executing on programmable computers each comprising at least one module component which comprises at least one processor (e.g. a microprocessor), a data storage system (including volatile and non-volatile memory and/or storage elements), at least one input device, and at least one output device. For example, and without limitation, the programmable computers (referred to here as computer systems) may be a personal computer, single board computer, laptop, personal data assistant, and cellular telephone, smart-phone device, tablet computer, and/or wireless device. Program code is applied to input data to perform the functions described herein and generate output information. The output information is applied to one or more output devices, in known fashion.

Each program is preferably implemented in a high level procedural or object oriented programming and/or scripting language to communicate with a computer system. However, the programs can be implemented in assembly or machine language, if desired. In any case, the language may be a compiled or interpreted language. Each such computer program is preferably stored on a storage media or a device (e.g. ROM or magnetic diskette) readable by a general or special purpose programmable computer, for configuring and operating the computer when the storage media or device is read by the computer to perform the procedures described herein. The subject system may also be considered to be implemented as a computer-readable storage medium, configured with a computer program, where the storage medium so configured causes a computer to operate in a specific and predefined manner to perform the functions described herein.

Specific examples and preferred embodiments are set out in the dependent claims.

### Description of embodiments

In the following detailed description, reference is made in sufficient detail to the above referenced principles, allowing those skilled in the art to practice the embodiments explained below.

As explained above, the system comprises among others, a processor and a memory. The memory comprises the model data (including the stationary and dynamic model data) that can be processed by the processor to produce or calculate a medical scene map. The calculation of the medical scene map is performed by the processor based on the model data stored in the memory for the set of data stream readings representing the positional parameters, and that are read from the interfaces from each movable component. The particular medical scene map that is produced for the actual positional parameters from the imaging modality components thus represents a virtual representation of the geometric state of the imaging modality, that is stored in the memory. This medical scene map is thus a virtual "copy" in memory of the actual geometric state of the imaging modality, and is based on the actual readings of the positional parameters for all components from the data streams.

The supervision function for the automated movement by an imaging modality is conceived as a continuous process whereby a calculated distance between a point on a surface of an object facing a distance detector and the detector mounting position is compared with the measured distance readings from said detector in the direction of said point on said surface of an object.

Since the mounting location of the distance detector on a surface of an object or modality component in the medical scene is known, it has to be comprised in the model data. The medical scene map, which then also comprises the location and orientation of the distance detector, will allow the calculation of any distance (calculated distance) between this distance detector mounting position and any point on an object surface.

The supervision process executed by the processor of the system thus simultaneously collects the distance measurement data (measured distance) and all positional parameters for a certain geometric state of the imaging modality. The positional parameter data are then used as input data for the model data and processed by the processor into a medical scene map. Based on this medical scene map, the calculated distances corresponding to the measured distances by the distance detector are calculated and compared with the measured distances. This process is continuously performed, and this at least while the modality components are moving.

As explained before, the medical scene map is a virtual representation of the actual state of the modality and its surroundings. Therefore, in order to assure that the virtual representation (in memory) matches the actual geometric state of the imaging modality, a distance detector can be positioned at a known location and orientation within the medical scene to validate the positions and orientations of observed or detected objects that are within its field-of-view or detection range. In case that the distance detector measures a perpendicular distance between its detector surface and a next object, this measured distance should match the equivalent calculated distance between the detector position stored in the medical scene map and the object from the medical scene map.

The comparison of the calculated distances versus the measured distances allow the evaluation of the proper functioning of the system, as each movement of the movable modality components will generate different measurement results for carefully positioned distance detectors. These different measurement results should in each case match the corresponding calculated distances from the medical scene map. Discrepancies between the measured distances and the calculated distances should be carefully compared within the tolerances of the distance measurement detectors. In case that structural deviations would occur, the supervision system should trigger the imaging modality allowing it to react appropriately. The trigger signal can then for instance alert a user, or for instance prevent further automated movement, or alternatively correct the movement course of the movable component. Structural deviations can occur when the assumed distance detector position and orientation does not match the actual detector position and orientation. In this case the distance detector position should be readjusted and calibrated to again match the measurement direction and position of the detector in the model data.

For better accuracy of the results, it is preferable that more than one distance is measured and compared with the calculated ones from the medical scene map. It is therefore preferable that more than one distance detector would be installed, and this preferably at carefully selected positions.

Further improvement of the technique can be achieved, when more distances can be measured at once for comparison when using detectors that can simultaneously measure multiple distances in different directions from the same position. The use of a 3D-camera or depth-camera, as well as depth scanners based on radar, microwaves or alike may be envisaged. In the case of the use of a depth-camera, the pixel values in an acquired depth image represent a value for the respective absolute distance between the detector and an object along the path of the respective pixel. This means that each depth image pixel can be considered as a distance measurement. Based on the medical scene map, a virtual depth-image can in principle be constructed providing a virtual depth view corresponding to the same acquisition geometry as the depth-camera. The virtual depth-image can then be compared with the acquired depth-image in order to validate the position and view of all involved objects or components.

Better results may also be achieved when carefully selecting the locations of the distance detectors. Ideally, the measured distances should vary when the modality state changes. In other words, it is preferable that the distance detector would be mounted on a movable component of the imaging modality, as this ensures that any movement of the modality component on which the detector is fixed would cause a different distance measurement. Moreover, the detection axis of the distance measurement should ideally be oriented towards other movable imaging modality components, such that again any movement of said movable components would cause changes in the measured distance by the distance detector. If the detector(s) are mounted on the surfaces of non-movable objects such as non-movable modality components or walls at least one moveable component shall be present within the field of view to again generate changing distances.

As explained above, the comparison of the expected distances versus the measured distances allow the evaluation of the proper functioning of the system, but at the same time could also indicate the presence of foreign objects or persons in the medical scene. In case that the measured distance from a distance detector does not match the anticipated and calculated distance of the medical scene map; there could be two causes: either the supervision system does not function properly as explained above, or the discrepancy is caused by the presence of an unidentified object (or person) that is not present in the medical scene map.

An unidentified object should be identified as such by the system in order to release the system for further use. This could be achieved in many ways; for instance, an operator could overrule the supervision system by confirming that the detected object is indeed an object that is not registered in the medical scene map. Or the supervision system could identify the object by confirming its location and presence by measuring distances with different detectors (and thus from different viewing angles). Another method is to identify and use the known background of the scene in case the unknown object does not fill the complete field of view and is moving. A further alternative is to observe the scene for a longer time period with the detector moving; the (static) unknown object should move consistently in the depth image.

The supervision system could bring automated modality movement even one step further by using the information about the presence, location and moving direction of unidentified objects to adjust the course of an automated modality movement during its movement. The supervision system should then detect the presence, position and moving direction of an object or person at a sufficiently high rate in order to allow to adjust the automated movements on-the-fly.

## Claims

1. System to supervise an automated movement of at least one movable component of an imaging modality within a medical scene to prevent collision between said movable component and other objects within said medical scene, said movable component providing a data stream comprising at least one positional parameter determining said movable components' geometric state in the medical scene,
the system comprising,
- an interface adapted to read out said positional parameter from said data stream from said movable component,
- a memory storing:
- dynamic model data representing geometric knowledge of said at least one movable component in the medical scene as a function of said positional parameter of said movable component in said data stream,
- stationary model data representing geometric knowledge on all non-movable objects in the medical scene,
- a medical scene map,
- at least one distance detector fixed at a known position in said medical scene, said known position is stored in said medical scene map, said distance detector providing at least one measured distance between said distance detector and an observed object along a detection axis of said distance detector,
- a processor configured to:
- calculate said medical scene map from said dynamic model data in combination with said at least one positional parameter, and from said stationary model data,
- calculate a calculated distance between said stored position of said distance detector in said medical scene map and a first object along said detection axis of said distance detector in said medical scene map,
- compare said at least one measured distance with said calculated distance, such that when the difference between said at least one measured distance and said calculated distance exceeds a threshold value, a trigger signal is sent to said imaging modality.

2. A system according to Claim 1, wherein said at least one distance detector is a 3D- or depth-camera.

3. A system according to Claim 1, wherein said at least one distance detector is a mm-wave radar with an array of transmitting and receiving antennas.

4. A system according to Claim 1, wherein said at least one distance detector is a phased array mm-wave radar.

5. A system according to Claim 1, wherein said at least one distance detector is an array of ultrasound transceivers including phased arrays.

6. A system according to Claim 1, wherein said other objects comprise movable and non-movable imaging modality components within said medical scene.

7. A system according to Claim 1, wherein said medical scene map is two-dimensional.

8. A system according to Claim 1, wherein said medical scene map is three-dimensional.

9. A system according to Claim 1, wherein said positional parameter is an angle between a joint connecting said movable component and another component.

10. A system according to Claim 1, wherein said positional parameter is a distance along which the movable component can extend in a predetermined direction.

11. A system according to Claim 1, wherein said known position of said distance detector is preferably on a surface of said movable component.

12. Method to supervise automated movement of at least one movable component of an imaging modality within a medical scene to prevent collision between said movable component and other objects within said medical scene, said movable component providing a data stream comprising at least one positional parameter determining said movable components' geometric state in the medical scene, comprising the steps of,
- reading out said positional parameter from said data stream from said movable component,
- storing in a memory dynamic model data representing geometric knowledge of said at least one movable component in the medical scene as a function of said positional parameter of said movable component in said data stream,
- storing in a memory stationary model data representing geometric knowledge on all non-movable objects in the medical scene,
- measuring a distance between a distance detector and an observed object along a detection axis of said distance detector, said distance detector being fixed at a known position in said medical scene, and storing said known position in said medical scene map,
- calculating said medical scene map from said dynamic model data in combination with said at least one positional parameter, and from said stationary model data, and storing said medical scene map in a memory,
- calculating a calculated distance between said stored position of said distance detector in said medical scene map and a first object along said detection axis of said distance detector in said medical scene map,
- comparing said at least one measured distance with said calculated distance, and sending a trigger signal to said imaging modality when the difference between said at least one measured distance and said calculated distance exceeds a threshold value.

## Patentansprüche

1. System zur Überwachung einer automatisierten Bewegung mindestens einer beweglichen Komponente einer Bildgebungsmodalität in einer medizinischen Szene, um ein Zusammenstoßen zwischen der beweglichen Komponente und weiteren Objekten in der medizinischen Szene zu verhindern, wobei die bewegliche Komponente einen Datenstrom liefert, der mindestens einen Positionsparameter, der den geometrischen Zustand der beweglichen Komponente in der medizinischen Szene bestimmt, umfasst,
wobei das System Folgendes umfasst:
- eine Schnittstelle, die so angepasst ist, dass sie den Positionsparameter aus dem Datenstrom der beweglichen Komponente ausliest,
- einen Speicher, der Folgendes speichert:
- Daten eines dynamischen Modells, die bekannte geometrische Kennzeichen der mindestens einen beweglichen Komponente in der medizinischen Szene als Funktion des Positionsparameters der beweglichen Komponente im Datenstrom darstellen,
- Daten eines stationären Modells, die bekannte geometrische Kennzeichen aller nicht-beweglichen Objekte in der medizinischen Szene darstellen,
- eine Abbildung der medizinischen Szene,
- mindestens einen Abstandsdetektor, der an einer bekannten Position in der medizinischen Szene befestigt ist, wobei die bekannte Position in der Abbildung der medizinischen Szene abgespeichert ist, wobei der Abstandsdetektor mindestens einen gemessenen Abstand zwischen dem Abstandsdetektor und einem wahrgenommenen Objekt entlang einer Detektionsachse des Abstandsdetektors liefert,
- einen Prozessor, der so konfiguriert ist, dass:
- er die Abbildung der medizinischen Szene aus den Daten des dynamischen Modells in Kombination mit dem mindestens einen Positionsparameter und aus den Daten des stationären Modells berechnet,
- er einen berechneten Abstand zwischen der abgespeicherten Position des Abstandsdetektors in der Abbildung der medizinischen Szene und einem ersten Objekt entlang der Detektionsachse des Abstandsdetektors in der Abbildung der medizinischen Szene berechnet,
- er den mindestens einen gemessenen Abstand mit dem berechneten Abstand vergleicht, wobei, wenn der Unterschied zwischen dem mindestens einen gemessenen Abstand und dem berechneten Abstand einen Schwellwert übersteigt, ein Triggersignal an die Bildgebungsmodalität übermittelt wird.

2. Ein System nach Anspruch 1, wobei der mindestens eine Abstandsdetektor eine 3D-Kamera oder Tiefenkamera ist.

3. Ein System nach Anspruch 1, wobei der mindestens eine Abstandsdetektor ein mm-Wellen-Radar mit einer Anordnung von Sende- und Empfangsantennen ist.

4. Ein System nach Anspruch 1, wobei der mindestens eine Abstandsdetektor ein mm-Wellen-Radar mit phasengesteuertem Feld ist.

5. Ein System nach Anspruch 1, wobei der mindestens eine Abstandsdetektor eine Anordnung von phasengesteuerte Felder umfassenden Ultraschall-Sender-Empfängern ist.

6. Ein System nach Anspruch 1, wobei die weiteren Objekte bewegliche und nicht-bewegliche Bildgebungsmodalitätskomponenten in der medizinischen Szene umfassen.

7. Ein System nach Anspruch 1, wobei die Abbildung der medizinischen Szene zweidimensional ist.

8. Ein System nach Anspruch 1, wobei die Abbildung der medizinischen Szene dreidimensional ist.

9. Ein System nach Anspruch 1, wobei der Positionsparameter ein Winkel zwischen einer die bewegliche Komponente und eine weitere Komponente verbindenden Verbindung ist.

10. Ein System nach Anspruch 1, wobei der Positionsparameter ein Abstand ist, über den sich die bewegliche Komponente in eine vorgegebene Richtung erstrecken kann.

11. Ein System nach Anspruch 1, wobei die bekannte Position des Abstandsdetektors bevorzugt auf einer Oberfläche der beweglichen Komponente vorliegt.

12. Verfahren zur Überwachung einer automatisierten Bewegung mindestens einer beweglichen Komponente einer Bildgebungsmodalität in einer medizinischen Szene, um ein Zusammenstoßen zwischen der beweglichen Komponente und weiteren Objekten in der medizinischen Szene zu verhindern, wobei die bewegliche Komponente einen Datenstrom liefert, der mindestens einen Positionsparameter, der den geometrischen Zustand der beweglichen Komponente in der medizinischen Szene bestimmt, umfasst, wobei das Verfahren folgende Schritte umfasst:
- Auslesen des Positionsparameters aus dem Datenstrom der beweglichen Komponente,
- Abspeichern, in einem Speicher, von Daten eines dynamischen Modells, die bekannte geometrische Kennzeichen der mindestens einen beweglichen Komponente in der medizinischen Szene als Funktion des Positionsparameters der beweglichen Komponente im Datenstrom darstellen,
- Abspeichern, in einem Speicher, von Daten eines stationären Modells, die bekannte geometrische Kennzeichen aller nicht-beweglichen Objekte in der medizinischen Szene darstellen,
- Messen eines Abstandes zwischen einem Abstandsdetektor und einem wahrgenommenen Objekt entlang einer Detektionsachse des Abstandsdetektors, wobei der Abstandsdetektor an einer bekannten Position in der medizinischen Szene befestigt ist, und Abspeichern der bekannten Position in der Abbildung der medizinischen Szene,
- Berechnen der Abbildung der medizinischen Szene aus den Daten des dynamischen Modells in Kombination mit dem mindestens einen Positionsparameter und aus den Daten des stationären Modells und Abspeichern der Abbildung der medizinischen Szene in einem Speicher,
- Berechnen eines berechneten Abstandes zwischen der abgespeicherten Position des Abstandsdetektors in der Abbildung der medizinischen Szene und einem ersten Objekt entlang der Detektionsachse des Abstandsdetektors in der Abbildung der medizinischen Szene,
- Vergleichen des mindestens einen gemessenen Abstandes mit dem berechneten Abstand und Übermitteln eines Triggersignals an die Bildgebungsmodalität, wenn der Unterschied zwischen dem mindestens einen gemessenen Abstand und dem berechneten Abstand einen Schwellwert übersteigt.

## Revendications

1. Système pour superviser un mouvement automatisé d'au moins un composant mobile d'une modalité d'imagerie dans une scène médicale afin de prévenir toute collision entre ledit composant mobile et d'autres objets dans ladite scène médicale, ledit composant mobile fournissant un flux de données comprenant au moins un paramètre positionnel déterminant l'état géométrique dudit composant mobile dans la scène médicale,
ledit système comprenant
- une interface adaptée de façon à lire ledit paramètre positionnel à partir dudit flux de données dudit composant mobile,
- une mémoire destinée à mémoriser:
- des données d'un modèle dynamique représentant des caractéristiques géométriques connues relatives audit au moins un composant mobile dans la scène médicale en fonction dudit paramètre positionnel dudit composant mobile dans ledit flux de données,
- des données d'un modèle stationnaire représentant des caractéristiques géométriques connues relatives à tous les objets non mobiles dans la scène médicale,
- une représentation mappée de la scène médicale,
- au moins un détecteur de distance fixé à une position connue dans ladite scène médicale, ladite position connue étant mémorisée dans ladite représentation mappée de la scène médicale, ledit détecteur de distance fournissant au moins une distance mesurée entre ledit détecteur de distance et un objet observé le long d'un axe de détection dudit détecteur de distance,
- un processeur configuré de façon à:
- calculer ladite représentation mappée de la scène médicale à partir desdites données du modèle dynamique en combinaison avec ledit au moins un paramètre positionnel et à partir desdites données du modèle stationnaire,
- calculer une distance calculée entre ladite position mémorisée dudit détecteur de distance dans ladite représentation mappée de la scène médicale et un premier objet le long dudit axe de détection dudit détecteur de distance dans ladite représentation mappée de la scène médicale,
- comparer ladite au moins une distance mesurée à ladite distance calculée de façon à ce que, lorsque la différence entre ladite au moins une distance mesurée et ladite distance calculée dépasse une valeur seuil, un signal de déclenchement soit transmis à ladite modalité d'imagerie.

2. Système selon la revendication 1, **caractérisé en ce que** ledit au moins un détecteur de distance est une caméra 3D ou une caméra de profondeur.

3. Système selon la revendication 1, **caractérisé en ce que** ledit au moins un détecteur de distance est un radar à ondes millimétriques comprenant un réseau d'antennes émettrices et réceptrices.

4. Système selon la revendication 1, **caractérisé en ce que** ledit au moins un détecteur de distance est un radar à ondes millimétriques à réseau à commande de phase.

5. Système selon la revendication 1, **caractérisé en ce que** ledit au moins un détecteur de distance est un réseau d'émetteurs-récepteurs à ultrasons comprenant des réseaux à commande de phase.

6. Système selon la revendication 1, **caractérisé en ce que** lesdits autres objets comprennent des composants mobiles et non mobiles de la modalité d'imagerie dans ladite scène médicale.

7. Système selon la revendication 1, **caractérisé en ce que** ladite représentation mappée de la scène médicale est bidimensionnelle.

8. Système selon la revendication 1, **caractérisé en ce que** ladite représentation mappée de la scène médicale est tridimensionnelle.

9. Système selon la revendication 1, **caractérisé en ce que** ledit paramètre positionnel est un angle entre une connexion reliant ledit composant mobile à un autre composant.

10. Système selon la revendication 1, **caractérisé en ce que** le paramètre positionnel est une distance le long de laquelle le composant mobile peut s'étendre dans un sens prédéfini.

11. Système selon la revendication 1, **caractérisé en ce que** ladite position connue dudit détecteur de distance se situe de préférence sur une surface dudit composant mobile.

12. Procédé pour superviser un mouvement automatisé d'au moins un composant mobile d'une modalité d'imagerie dans une scène médicale afin de prévenir toute collision entre ledit composant mobile et d'autres objets dans ladite scène médicale, ledit composant mobile fournissant un flux de données comprenant au moins un paramètre positionnel déterminant l'état géométrique dudit composant mobile dans la scène médicale, ledit procédé comprenant les étapes consistant à
- lire ledit paramètre positionnel à partir dudit flux de données dudit composant mobile,
- mémoriser, dans une mémoire, des données d'un modèle dynamique représentant des caractéristiques géométriques connues relatives audit au moins un composant mobile dans la scène médicale en fonction du paramètre positionnel dudit composant mobile dans ledit flux de données,
- mémoriser, dans une mémoire, des données d'un modèle stationnaire représentant des caractéristiques géométriques connues relatives à tous les objets non mobiles dans la scène médicale,
- mesurer une distance entre un détecteur de distance et un objet observé le long d'un axe de détection dudit détecteur de distance, ledit détecteur de distance étant fixé à une position connue dans ladite scène médicale, et mémoriser ladite position connue dans ladite représentation mappée de la scène médicale,
- calculer ladite représentation mappée de la scène médicale à partir desdites données du modèle dynamique en combinaison avec ledit au moins un paramètre positionnel et à partir desdites données du modèle stationnaire et mémoriser ladite représentation mappée de la scène médicale dans une mémoire,
- calculer une distance calculée entre ladite position mémorisée dudit détecteur de distance dans ladite représentation mappée de la scène médicale et un premier objet le long dudit axe de détection dudit détecteur de distance dans ladite représentation mappée de la scène médicale,
- comparer ladite au moins une distance mesurée à ladite distance calculée et transmettre un signal de déclenchement à ladite modalité d'imagerie lorsque la différence entre ladite au moins une distance mesurée et ladite distance calculée dépasse une valeur seuil.
